# EUROPEAN PATENT APPLICATION

(11) **EP 1 857 435 A2**
(43) Date of publication of application: **21.11.2007**
(21) Application number: 07009205.1
(22) Date of filing: 07.05.2007
(51) Int. Cl.: C07C 213/00, C07C 213/08, C07C 217/52, C07C 217/54, C07D 233/20, A61K 31/381

(54) **A process for the preparation of aryloxypropylamines**

(30) Priority: 18.05.2006 IT MI20060984
(71) Applicant: Dipharma Francis S.r.l., 20021 Baranzate di Bollate MI (IT)
(72) Inventor: Mantegazza, Simone, 20144 Milano (IT); Razzetti, Gabriele, 20099 Sesto San Giovanni (MI) (IT); Rasparini, Marcello, 27010 Cura Carpignano (PV) (IT); Rossi, Roberto, 27100 Pavia (IT); Allegrini, Pietro, 20097 San Donato Milanese (MI) (IT)
(74) Representative: Minoja, Fabrizio

(57) **Abstract**

A process for the preparation of a compound of formula (I), or a salt thereof, both as the as individual isomer and as mixtures thereof, 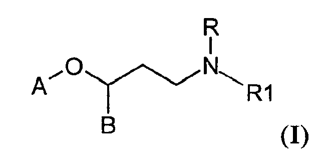 wherein each of A and B is independently aryl or heteroaryl optionally substituted with 1 to 4 substituents; and R and R₁, which can be the same or different, are hydrogen, C₁-C₆ alkyl or an amino-protecting group; comprising the reaction of a compound (II) 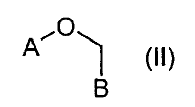 wherein A and B are as defined above, with a compound (III) 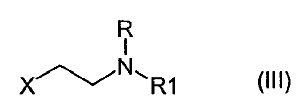 wherein each of R and R₁ is independently C₁-C₆ alkyl or an amino-protecting group; and X is a leaving group; in the presence of a basic agent; and, if desired the conversion of a compound (I) to another compound (I); and/or, if desired, the separation of an isomeric mixture of a compound (I) into the individual isomers; and/or, if desired, the conversion of a compound (I) to a salt thereof.

## Description

### FIELD OF THE INVENTION

The present invention relates to a process for the preparation of a compound of formula (I), or a salt thereof, both as the individual isomer and the isomeric mixture 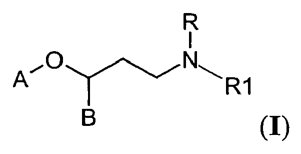 wherein each of A and B is independently optionally substituted aryl or heteroaryl, as herein defined; R and R₁, which can be the same or different, are hydrogen, C₁-C₆ alkyl or an amino-protecting group. The compounds of formula (I) are useful as medicaments, in particular those in which R is hydrogen and R₁ is methyl are useful as antidepressants.

### TECHNOLOGICAL BACKGROUND

The compounds of formula (I) belong to the "oxetine" family, and are antidepressants and inhibitors of synaptic readsorption of neuronal mediators such as serotonin and noradrenalin. Examples of said "oxetines" are duloxetine, atomoxetine, fluoxetine and nisoxetine. A number of synthetic methods for the preparation of these compounds are known. Many of them, however, make use of expensive, hardly available starting products; moreover their synthesis requires many steps, which make the process troublesome. Examples of said synthetic methods are disclosed in US 5,023,269; US 5,362,886 and EP 457,559 for duloxetine and US 4,018,895 for fluoxetine or atomoxetine.

There is therefore the need for an alternative process for the preparation of said compounds, using easily available or easy-to-prepare starting products as well as operative conditions suited to the industrial production, so as to reduce costs.

### SUMMARY OF THE INVENTION

The invention relates to a process for the preparation of a compound of formula (I), or a salt thereof, both as the individual isomer and the isomeric mixture 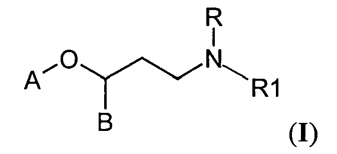 wherein each of A and B is independently optionally substituted aryl or heteroaryl, as herein defined; R and R₁, which can be the same or different, are hydrogen, C₁-C₆ alkyl or an amino-protecting group; comprising the reaction of a compound of formula (II) with a compound of formula (III) 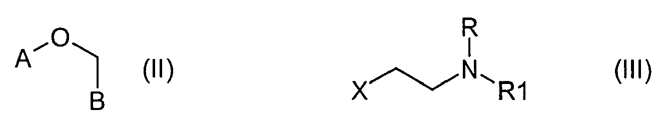 wherein A and B are as herein defined, R₁ and R₂ are independently C₁-C₆ alkyl or an amino-protecting group; and X is a leaving group;
in the presence of a basic agent.

The process of the invention overcomes the problems mentioned above.

### DETAILED DISCLOSURE OF THE INVENTION

The object of the invention is a process for the preparation of a compound of formula (I), or a salt thereof, both as the individual isomer or isomeric mixtures thereof 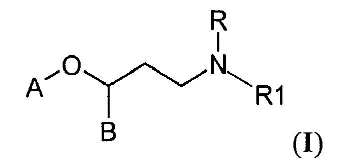 wherein each of A and B is independently aryl or heteroaryl optionally substituted by 1 to 4 substituents, which may be the same or different, selected from halogen, nitro, cyano, C₁-C₆ alkyl optionally substituted by halogen, C₁-C₆ alkylthio, C₁-C₆ alkoxy, and -N(RₐR_{b}) wherein Rₐ and R_{b}, which may be the same or different, are C₁-C₆ alkyl, or Rₐ and R_{b}, taken with the nitrogen atom they are linked to, form a 5- or 6- membered saturated or unsaturated ring, optionally containing 1, 2 or 3 further heteroatoms selected from nitrogen and oxygen; and
R and R₁, which may be the same or different, are hydrogen, C₁-C₆ alkyl or an amino-protective group;
comprising the reaction of a compound (II) 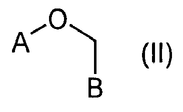 wherein A and B are as defined above,
with a compound (III) 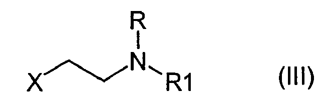 wherein each of R and R₁ is independently C₁-C₆ alkyl or an amino-protecting group; and X is a leaving group;
in the presence of a basic agent; and, if desired the conversion of a compound (I) to another compound (I); and/or, if desired, the separation of an isomeric mixture of a compound (I) into the individual isomers; and/or, if desired, the conversion of a compound (I) to a salt thereof.

An isomer of a compound (I) may be for example a geometrical or optical isomer, preferably an (R) or (S) enantiomer.

A salt of a compound (I) is for example a salt with an acid or a base, preferably a pharmaceutically acceptable salt, in particular hydrochloride, hydrobromide, sulfate, mandelate, tartrate, dibenzoyl-tartrate, ditoluyl-tartrate, oxalate, mesylate, maleate, preferably mandelate, hydrochloride and oxalate.

When A and/or B are aryl, e.g. phenyl or naphthyl, A is preferably phenyl or naphthyl, optionally substituted with C₁-C₄ alkyl, in turn optionally substituted with halogen; or with C₁-C₄ alkoxy. B as aryl is preferably phenyl.

A substituent A or B, as heteroaryl, is for example a heterocyclic ring, typically monocyclic or bicyclic, unsaturated, optionally containing 1, 2 or 3 heteroatoms selected from nitrogen, oxygen and sulfur. Examples of said heterocycle are thiophene, furan, pyrrole, pyridine, pyrazine, quinoline, indole, thionaphthene, benzofuran, quinoxaline and phthalazine. In particular B as heteroaryl is preferably thienyl.

A halogen atom is for example fluorine, chlorine, bromine or iodine; in particular fluorine or chlorine.

A C₁-C₆ alkyl group is typically a C₁-C₄ alkyl group, e.g. methyl, ethyl, propyl, isopropyl, n-butyl, sec-butyl or tert-butyl, in particular methyl, ethyl, propyl or isopropyl. When this is substituted by halogen, it may be substituted by 1 to 3 halogen atoms, as defined above; and is preferably trifluoromethyl.

A C₁-C₆ alkylthio group is typically a C₁-C₄ alkylthio group, in particular methylthio or ethylthio.

A C₁-C₆ alkoxy group is typically a C₁-C₄ alkoxy group, in particular methoxy, ethoxy and propoxy.

A -N(RₐR_{b}) group is for example a dimethylamino, methyl-ethylamino, diethylamino or diisopropylamino group.

When a -N(RₐR_{b}) group forms a 5- or 6- membered ring, as defined above, this is for example a pyrrolidino, piperidino, piperazino, morpholino, pyrrolyl, imidazolyl or pyrazolyl residue.

An amino-protected group can be an amino group protected with one of the protecting groups conventionally used in peptide chemistry. Typically an amino group is protected as the carbamate, amide, imide or enamine, in particular, as the ethyl carbamate, phenyl carbamate, acetamide and phthalimide.

A leaving group X may be for example a halogen atom, preferably chlorine or bromine, in particular bromine; or a hydroxy group activated by esterification, for example through an alkanesulfonyloxy group, typically mesyloxy, or an arylsulfonyloxy group, typically tosyloxy, or a perfluoroalkanesulfonyloxy group, such as trifluoromethanesulfonyloxy and nonafluorobutanesulfonyloxy; preferably chlorine, bromine, iodine, methanesulfonyloxy, p-toluenesulfonyloxy; more preferably chlorine.

A basic agent is for example a compound of formula E-M, wherein M is an alkali or alkaline-earth metal, such as sodium, lithium, potassium or magnesium and E is a strong organic or inorganic base, such as a hydride, an alkoxide, an alkyl carbanion, a silyl anion or an amide anion; or a compound of formula R_{c}-MgY or (R_{c})₂Mg wherein R_{c} is C₁-C₆ alkyl, C₅-C₇ cycloalkyl, aryl or aryl-C₁-C₆ alkyl and Y is a halogen, or a -N(RₐR_{b}) group as defined above.

A C₁-C₆ alkyl or aryl group is as defined above.

Y as halogen may be chlorine, bromine or iodine, preferably chlorine or bromine.

A C₁-C₆ aryl-alkyl group is preferably a C₁-C₄ aryl-alkyl group, in particular benzyl or phenylethyl.

A C₅-C₇ cycloalkyl group is typically a C₅-C₆ cycloalkyl group, preferably cyclohexyl.

Preferred examples of basic agent are butyl-lithium, hexyl-lithium, t-butyl-lithium, phenyl lithium, ethyl-magnesium bromide, cyclohexyl-magnesium chloride, benzyl-magnesium bromide, ethyl-magnesium-diisopropylamide, dibutyl-magnesium, magnesium diisopropylamide, lithium diisopropylamide, lithium bis-trimethylsilylamide, lithium tetramethylpiperidide, potassium tert-butoxide, sodium or potassium hydride, more preferably butyl lithium, hexyl lithium and lithium diisopropylamide.

The reaction between a compound (II) and a compound (III) can be carried out in the presence of a solvent, preferably an anhydrous organic solvent, typically an aliphatic or aromatic hydrocarbon, e.g hexane, toluene; petroleum ether; an ether, e.g tetrahydrofuran, dioxane, diethyl ether, methyl-tert-butyl ether; or a mixture of two or more, preferably two or three, of said solvents. The reaction is preferably carried out in an ether solvent, in particular tetrahydrofuran or methyl-tert-butyl ether.

The stoichiometric ratio of a compound (II) to the basic agent approx. ranges from 0.5 to 10, preferably approx. from 1 to 3. The reaction can be carried out at a temperature approx. ranging from -80°C to 10°C, preferably from -15°C to 0°C.

When the basic agent is a compound of formula E-M, as defined above, the reaction can be carried out in the presence of a ligand, typically a polyamine, in particular a diamine chosen for example from tetramethylethylendiamine, pentamethyldiethylentriamine and trans-tetramethylcycloexantriamine, preferably tetramethylethylendiamine.

The conversion of a compound (I) into another compound (I), the separation of an isomeric mixture into the individual isomers, as well as the conversion of a compound (I) to a salt thereof can be carried out according to known methods.

For example a compound (I) in which R is an amino-protecting group can be converted to a compound (I) in which R is hydrogen by removing the protecting group according to known methods, e.g. hydrolysis.

A compound in which R is a methyl group can be converted to a compound of formula (I) in which R is an amino-protecting group by means of a demethylation reaction according to known methods, for example with chloroformates as disclosed in EP 457559 for the preparation of duloxetine.

Finally, if desired, the product can be resolved into its enantiomers according to known methods, such as those described for atomoxetine in EP 52492.

Preferred compounds of formula (I) are those wherein:
A is phenyl or naphthyl optionally substituted with C₁-C₄ alkyl in turn optionally substituted with halogen; or with C₁-C₄ alkoxy;
B is phenyl or thienyl;
one of R and R₁ is C₁-C₄ alkyl and the other is hydrogen, C₁-C₄ alkyl, or an amino-protecting group; and the salts thereof.

Specific examples of compounds of formula (I) are:
- (+)-N-methyl-3-(1-naphthalenyloxy)-3-(2-thienyl)propanamine (duloxetine);
- N,N-dimethyl-3-(1-naphthalenyloxy)-3-(2-thienyl)propanamine;
- (-)-N-methyl-3-(2-methyl-phenyloxy)-3-phenyl-propanamine (atomoxetine);
- N,N-dimethyl-3-(2-methyl-phenyloxy)-3-phenyl-propanamine;
- N-methyl-3-(4-trifluoromethyl-phenyloxy)-3-phenyl-propanamine (fluoxetine);
- N,N-dimethyl-3-(4-trifluoromethyl-phenyloxy)-3-phenyl-propanamine;
- N-methyl-3-(4-methoxy-phenyloxy)-3-phenyl-propanamine (nisoxetine);
- N,N-dimethyl-3-(4-methoxy-phenyloxy)-3-phenyl-propanamine;
- phenyl N-methyl-3-(2-methyl-phenyloxy)-3-phenyl-propylcarbamate;
- phenyl N-methyl-3-(4-trifluoromethyl-phenyloxy)-3-phenyl-propylcarbamate;
- phenyl N-methyl-3-(1-naphthalenyloxy)-3-(2-thienyl)-propylcarbamate;
- phenyl N-methyl-3-(4-methoxy-phenyloxy)-3-phenyl-propylcarbamate;
and the salts thereof.

A compound (II), which can optionally be isolated, can be obtained by reaction between a compound (IV), or a salt thereof, with a compound (V), wherein A, B and X are as defined above. In particular X is Br or Cl.

The reaction, which can thus be schematized 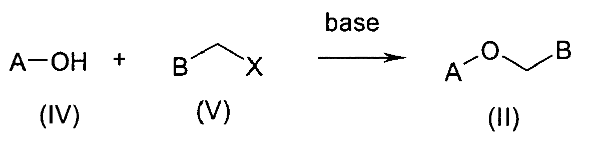 is preferably carried out in the presence of a basic agent. Examples of said agent are sodium, potassium, lithium or calcium hydroxide; sodium or potassium carbonate; a tertiary organic amine such as triethylamine, diisopropylethylamine or an alkali alkoxide such as sodium methoxide or sodium ethoxide. Preferably, the basic agent is sodium or potassium hydroxide, or sodium or potassium carbonate.

The stoichiometric ratio of compound (V) to compound (IV), or a salt thereof, approximately ranges from 0.5 to 10. preferably approximately from 1 to 1.5.

The reaction can optionally be carried out in the presence of a solvent. The solvent may typically be an organic solvent such as a ketone, e.g. acetone, diethyl ketone, methyl-isobutylketone; an ether, e.g. tetrahydrofuran, dioxane, diethyl ether; a chlorinated solvent, e.g. dichloromethane, dichloroethane, tetrachloroethylene, chlorobenzene or dichlorobenzene; an alkanol, e.g. methanol, ethanol or isopropanol, or a mixture of two or more, in particular of two or three, of said solvents optionally with water. The reaction is preferably carried out in a keto solvent, in particular acetone or an acetone/water mixture.

The reaction can be carried out at a temperature approx. ranging from 0°C to the reflux temperature of the reaction mixture, preferably approx. from 25°C to 50°C.

The following examples illustrate the invention.

### Example 1

### N,N-Dimethyl-3-(1-naphthalenyloxy)-3-(2-thienyl)-propanamine

A solution of 2-(naphthalen-1-yloxymethyl)-thiophene (0.89 g, 3.71 mmoles) in tetrahydrofuran (2 ml), kept under magnetic stirring and inert atmosphere at a temperature of about -70°C, is added with a solution of lithium diisopropylamide (3.71 mmoles, 1.4 M in tetrahydrofuran), previously prepared.

The mixture is reacted for about 30', then a solution of dimethylaminoethyl chloride (0.44 g, 4.10 mmoles) is dropped therein, keeping a temperature ranging from -50°C to -20°C. After completion of the addition, the reaction mixture is kept at about 20°C for 14 hours. Afterwards, 10 ml of H_{2O} are added. The aqueous phase is extracted twice with methyl-tert-butyl ether. The organic phase is separated, dried over anhydrous sodium sulfate, filtered through Celite and distilled under reduced pressure to a residue. The product is isolated by silica gel flash chromatography. (Ethyl acetate:Methanol: 2-Propanol=5:4:1)

¹H NMR (300 MHz, CDCl₃) δ: 2.17-2.32 m, 2H; 2.26 s, 6H; 2.38-2.58 m, 2H; 5.78 dd, 1H; 6.89 d, 1H; 6.93 dd, 1H; 7.05 d, 1H; 7.19 d, 1H; 7.25 dd, 1H; 7.37 d, 1H; 7.42-7.50 m, 2H; 7.62-7.80 m, 1H; 8.21-8.38 m, 1H.

### Example 2

### N,N-Dimethyl-3-(2-methylphenyloxy)-3-phenyl-propanamine

A three-necked reactor equipped with mechanical stirrer, thermometer, under nitrogen stream is loaded with approx. 95% o-tolyl-benzyl ether (30.0 g; 0.144 mol), tetramethylethylenediamine (20.0 g, 0.173 mol), methyl-tert-butyl ether (120 ml) and cooled to a temperature approx. ranging from -15°C to -20°C. A 2:3 M hexyl-lithium solution (68.8 mL, 0.158 mol) in hexane is slowly added keeping the temperature below -10°C. After completion of the addition, the mixture is reacted for about 15-20 minutes at approx. -10°C.

A solution of 2-dimethylamminoethyl chloride (24.8 g, 0.173 mol) in methyl-tert-butyl ether (60 mL) is prepared and dropped into the above solution, keeping the temperature below 0-5°C. The mixture is reacted at a temperature of about 10°C for approx. 1-2 hours, then water (100 ml) is added to quench the reaction. The phases are separated and washing with water (100 ml) is repeated. A solution of oxalic acid (16.3 g, 0.173 mol) in isopropanol (60 ml) is slowly dropped into the organic phase at a temperature of about 40-45°C, keeping this temperature for approx. 30 minutes, then the mixture is left to spontaneously cool at room temperature. The resulting solid is filtered, washed with a methyl-tert-butyl ether and isopropanol 1:1 mixture, then dried overnight in a static dryer at about 45°C under vacuum. 40.4 g of product are obtained, 78% yield.

¹H NMR (300 MHz, DMSO-d6) δ (ppm): 7.40-7.22 (m, 5H); 7.10 (d, 1H); 6.95 (t, 1H); 6.76-6.66 (m, 2H); 5.80 (m, 1H); 3.20-3.00 (m, 2H); 2.70 (s, 3H); 2.60 (s, 3H); 2.25 (s, 3H); 2.40-2.10 (m, 2H).

By proceeding analogously, the following compounds can be obtained:
- N,N-dimethyl-3-(4-trifluoromethyl-phenyloxy)-3-phenyl-propanamine; and
- N,N-dimethyl-3-(4-methoxy-phenyloxy)-3-phenyl-propanamine.

### Example 3

### O-Tolyl-benzyl ether

A three-necked round-bottom flask equipped with condenser, magnetic stirrer, thermometer, is loaded with o-cresol (100 g; 0.925 mol), potassium iodide (7.7 g; 0.046 mol) and acetone (650 ml) under nitrogen stream, then sodium carbonate (166 g; 1.20 mol) is added, keeping the suspension stirred. The resulting mixture is then refluxed while dropping benzyl chloride (127 g; 1.00 mol) therein in 30 minutes. After 18 hours, the mixture is concentrated under reduced pressure to a residue, added with water (400 ml) and toluene (200 ml), then heated to dissolution of the salts.
The phases are separated and the organic phase is washed with water, dried over sodium sulfate and evaporated to a residue. 156.1 g of o-tolyl-benzyl ether as an oil are obtained.

### Example 4

### Phenyl N-methyl-3-(2-methyl-phenyloxy)-3-phenyl-propylcarbamate

A three-necked reactor equipped with mechanical stirrer, thermometer, under nitrogen stream, is loaded with N,N-dimethyl-(3-phenyl-3-o-tolyloxy-propyl)-amine (11.9 g, 0.0441 mol) and toluene (50 ml) and refluxed. Phenyl chloroformate (7.6 g, 0.0486 mol) is slowly dropped therein in about 30 minutes keeping the reflux temperature for approx. 1-2 hours. The reaction mixture is cooled at room temperature under stirring for 16 hours, then diluted with dichloromethane (100 ml) and the organic phase is washed with a 10% sodium hydroxide solution (50 ml). The phases are separated and the aqueous phase is back-extracted with dichloromethane (100 ml). The combined organic phases are washed with a 1N hydrochloric acid solution (50 ml), then a sodium chloride saturated solution (50 ml). The organic phase is concentrated under reduced pressure to obtain 15.8 g of an oil.

¹H NMR (300 MHz, CDCl₃) δ (ppm) signals doubled by amido isomery: 7.40-7.05 (m, 10H); 7.00-6.90 (m, 2H); 6.80 (t, 1H); 6.60 (t, 1H); 5.25 (m, 1H); 3.90-3.50 (m, 2H); 3.05 (m, 3H); 2.40-2.20 (m, 5H).

By proceeding analogously, the following compounds can be obtained:
- Phenyl N-methyl-3-(4-trifluoromethyl-phenyloxy)-3-phenyl-propylcarbamate;
- Phenyl N-methyl-3-(1-naphthalenyloxy)-3-(2-thienyl)-propylcarbamate; and
- Phenyl N-methyl-3-(4-methoxy-phenyloxy)-3-phenyl-propylcarbamate.

### Example 5

### N-Methyl-3-(2-methylphenyloxy)-3-phenyl-propanamine

A three-necked reactor equipped with mechanical stirrer, thermometer, under nitrogen stream is loaded with phenyl methyl-(3-phenyl-3-o-tolyloxy-propyl)carbamate (15.8 g, 0.0441 mol), 1-butanol (50 ml), and heated to a temperature of 50-60°C, then added with potassium hydroxide (8.20 g, 0.132 mol) and refluxed for 16h. The mixture is cooled at room temperature and diluted with water (50 ml). The phases are separated, the organic phase is diluted with toluene (50 ml), washed 3 times with water (25 ml), then concentrated under reduced pressure, thereby obtaining an oil, 9.30 g, 82% yield.

¹H NMR (300 MHz, CDC1₃) δ (ppm): 7.35-7.25 (m, 5H); 7.15 (d, 1H); 6.95 (t, 1H); 6.80 (t, 1H); 6.65 (d, 1H); 5.30 (dd, 1H); 2.80 (t, 2H); 2.45 (s, 3H); 2.35 (s, 3H); 2.20 (m, 1H); 2.05 (m, 1H).

By proceeding analogously, the following compounds can be obtained:
- N-Methyl-3-(4-trifluoromethyl-phenyloxy)-3-phenyl-propanamine;
- N-Methyl-3-(1-naphthalenyloxy)-3-(2-thienyl)-propanamine; and
- N-Methyl-3-(4-methoxy-phenyloxy)-3-phenyl-propanamine.
If desired, the compounds N-methyl-3-(2-methylphenyloxy)-3-phenyl-propanamine and N-methyl-3-(1-naphthalenyloxy)-3-(2-thienyl)-propanamine can be separated into the single enantiomers, according to known methods, to obtain (-)-N-methyl-3-(2-methylphenyloxy)-3-phenyl-propanamine and (+)-N-methyl-3-(1-naphthalenyloxy)-3-(2-thienyl)-propanamine, respectively.

### Example 6

### N,N-Dimethyl-3-(2-methylphenyloxy)-3-phenyl-propanamine

A three-necked reactor equipped with mechanical stirrer, thermometer, under nitrogen stream is loaded with approx. 95% o-tolyl-benzyl ether (30.0 g; 0.144 mol), tetramethylethylenediamine (20.0 g, 0.173 mol), methyl-tert-butyl ether (120 ml), and cooled to a temperature approx. ranging from -15°C to -20°C. A 2.5 M butyl-lithium solution (63.2 mL, 0.158 mol) in hexane is slowly added keeping the temperature below -10°C. After completion of the addition, the mixture is reacted for about 15-20 minutes at approx. -10°C.

A solution of 2-dimethylamminoethyl chloride (24.8 g, 0.173 mol) in methyl-tert-butyl ether (60 mL) is prepared and dropped into the above solution, keeping the temperature below approx. 0-5°C. The mixture is reacted at a temperature of about 10°C for approx. 1-2 hours, then water is added (100 ml) to quench the reaction. The phases are separated and washing with water (100 ml) is repeated. The organic phase is slowly dropwise added with an oxalic acid (16.3 g, 0.173 mol) solution in isopropanol (60 ml) at a temperature of about 40-45°C. This temperature is kept for approx. 30 minutes, then the mixture is left to spontaneously cool at room temperature. The resulting solid is filtered and washed with a methyl-tert-butyl ether and isopropanol 1:1 mixture, then dried in a static dryer at about 45°C under vacuum overnight. 40.4 g of product are obtained, 78% yield.

## Claims

1. A process for the preparation of a compound of formula (I), or a salt thereof, as the individual isomer or a mixture thereof, 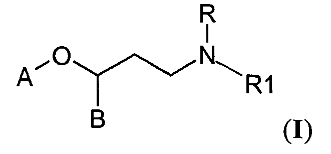 wherein each of A and B is independently aryl or heteroaryl optionally substituted by 1 to 4 substituents, which can be the same or different, selected from halogen, nitro, cyano, C₁-C₆ alkyl optionally substituted with halogen, C₁-C₆ alkylthio, C₁-C₆ alkoxy, and -N(RₐR_{b}) wherein Rₐ and R_{b}, which can be the same or different, are C₁-C₆ alkyl, or Rₐ and R_{b}, taken with the nitrogen atom they are linked to, form a 5- or 6- membered saturated or unsaturated ring, optionally containing 1, 2 or 3 further heteroatoms selected from nitrogen and oxygen; and
R and R₁, which can be the same or different, are hydrogen, C₁-C₆ alkyl or an amino-protecting group;
comprising the reaction of a compound (II) 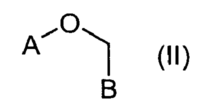 wherein A and B are as defined above,
with a compound (III) 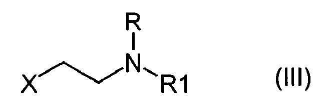 wherein each of R and R₁ is independently C₁-C₆ alkyl or an amino-protecting group; and X is a leaving group;
in the presence of a basic agent; and, if desired the conversion of a compound (I) to another compound (I); and/or, if desired, the separation of an isomeric mixture of a compound (I) into the individual isomers; and/or, if desired, the conversion of a compound (I) to a salt thereof.

2. A process as claimed in to claim 1, wherein in a compound of formula (I), or in a salt thereof,
A is phenyl or naphthyl optionally substituted with C₁-C₄ alkyl in turn optionally substituted with halogen; or with C₁-C₄ alkoxy;
B is phenyl or thienyl;
one of R and R₁ is C₁-C₄ alkyl and the other is hydrogen, C₁-C₄ alkyl, or an amino-protecting group.

3. A process as claimed in claim 1, wherein the compound of formula (I) is selected from:
• (+)-N-methyl-3-(1-naphthalenyloxy)-3-(2-thienyl)propanamine;
• N,N-dimethyl-3-(1-naphthalenyloxy)-3-(2-thienyl)propanamine;
• (-)-N-methyl-3-(2-methyl-phenyloxy)-3-phenyl-propanamine;
• N,N-dimethyl-3-(2-methyl-phenyloxy)-3-phenyl-propanamine;
• N-methyl-3-(4-trifluoromethyl-phenyloxy)-3-phenyl-propanamine;
• N,N-dimethyl-3-(4-trifluoromethyl-phenyloxy)-3-phenyl-propanamine;
• N-methyl-3-(4-methoxy-phenyloxy)-3-phenyl-propanamine;
• N,N-dimethyl-3-(4-methoxy-phenyloxy)-3-phenyl-propanamine;
• phenyl N-methyl-3-(2-methyl-phenyloxy)-3-phenyl-propylcarbamate;
• phenyl N-methyl-3-(4-trifluoromethyl-phenyloxy)-3-phenyl-propylcarbamate;
• phenyl N-methyl-3-(1-naphthalenyloxy)-3-(2-thienyl)-propylcarbamate;
• phenyl N-methyl-3-(4-methoxy-phenyloxy)-3-phenyl-propylcarbamate;
or a salt thereof.

4. A process as claimed in claim 1, wherein the basic agent is a compound of formula E-M, wherein M is an alkali or alkaline-earth metal and E is a strong organic or inorganic base; or a compound of formula R_{c}-MgY or of formula (R_{c})₂Mg wherein R_{c} is C₁-C₆ alkyl, C₅-C₇ cycloalkyl, aryl or aryl-C₁-C₆ alkyl and Y is a halogen or a group -N(RₐR_{b}) as defined in claim 1.

5. A process as claimed in claim 4, wherein the basic agent is selected from butyl-lithium, hexyl-lithium, t-butyl-lithium, phenyl lithium, ethyl-magnesium bromide, cyclohexyl-magnesium chloride, benzyl-magnesium bromide, ethyl-magnesium-diisopropylamide, dibutyl-magnesium, magnesium diisopropylamide, lithium diisopropylamide, lithium bis-trimethylsilylamide, lithium tetramethylpiperidide, potassium tert-butoxide, and sodium or potassium hydride.

6. A process as claimed in claim 5, wherein the basic agent is selected from butyl lithium, hexyl lithium and lithium diisopropylamide.

7. A process according to claims 4 to 6, wherein the reaction is carried out in the presence of a ligand.

8. A process as claimed in claim 1, wherein the leaving group is a halogen atom or a hydroxy group activated by esterification.

9. A process as claimed in claim 1, wherein the reaction is carried out in an anhydrous organic solvent.

10. A process as claimed in claim 9, wherein the solvent is selected from an aliphatic or aromatic hydrocarbon, petroleum ether; an ether; or a mixture of two or more than said solvents.

11. A process as claimed in claim 1, wherein the stoichiometric of compound of formula (II) to basic agent approx. ranges from 0.5 to 10.
